# EUROPÄISCHE PATENTSCHRIFT

(11) **EP 0 747 340 B1**
(45) Veröffentlichungstag und Bekanntmachung des Hinweises auf die Patenterteilung: **01.09.1999**
(21) Anmeldenummer: 96890094.4
(22) Anmeldetag: 04.06.1996
(51) Int. Cl.: C07C 51/42, C07C 59/265

(54) **Wasserfreies Trinatriumcitrat, Verfahren zu seiner Herstellung und seine Verwendung als Trägermaterial**
Anhydrous trisodiumcitrate, process for its preparation and its use as carriers
Citrate trisodique anhydre, procédé pour sa préparation et son utilisation comme support

(30) Priorität: 06.06.1995 AT 95995
(43) Veröffentlichungstag der Anmeldung: 11.12.1996
(73) Patentinhaber: Jungbunzlauer Ges.m.b.H., 1010 Wien (AT)
(72) Erfinder: Kirkovits, August Ernst, Dipl. Ing., A-2153 Stronegg 21 (AT); Gass, Josef, Dr., 2265 Drösing (AT)
(74) Vertreter: Collin, Hans, Dipl.-Ing. Dr.

(56) Entgegenhaltungen:
- EP-A- 0 479 084
- DE-A- 2 305 149
- STN-INFORMATION SERVICE, FILE: REG, XP002013569 "RN= 68-04-2: Sodium citrate anhydrous"

## Beschreibung

Die Erfindung betrifft wasserfreies Trinatriumcitrat mit gegenüber einer kristallinen Hydratform unveränderter Struktur, und dieses Material als Träger von anorganischen und/oder organischen Substanzen, insbesondere nach Tränken mit Flüssigkeiten.

Trinatriumcitrat wird durch Neutralisieren von Zitronensäure mit Natronlauge und Kristallisierenlassen in Hydratform erhalten, wobei mehrere Hydratformen bekannt sind und das Dihydrat die technisch bedeutsamste Form darstellt.

Trinatriumcitrat-Dihydrat wird in der Lebensmittelindustrie als pH-Regulator, als Komplexbildner, Stabilisator, Synergist für Antioxidation und als Käsereisalz, um nur einige Anwendungen zu nennen, eingesetzt. Durch die zunehmenden Forderungen nach umweltverträglichen Waschmitteln wird Trinatriumcitrat-Dihydrat seit einiger Zeit bei phosphatfreien Waschmitteln als Builder eingesetzt. Seine Wirkung ist, die Kalkabscheidung an der Wäsche zu verhindern, die Verstärkung von Tensiden, die Einstellung der Alkalinität der Waschflotte und die Verbesserung der Pulvereigenschaften.

Nunmehr wurde gefunden, daß man kristalline Trinatriumcitrathydrate durch Erwärmen zu wasserfreien Produkten entwässern kann, deren Struktur gegenüber dem Ausgangshydrat unverändert ist, mit anderen Worten zu Produkten, die porös sind und daher eine innere Oberfläche aufweisen, wobei bei Verwendung von Dihydrat als Ausgangsmaterial die Porosität des Produkts dann etwa 15 % ausmacht.

Diese Produkte behalten ihre Rieselfähigkeit und Fließeigenschaften über einen langen Zeitraum bei, zeigen gegenüber der Hydratform weitaus kürzere Auflösezeiten und lassen sich mit jedem anderen Produkt vermischen, d.h. sowohl mit Trockensubstanzen oder Flüssigkeiten, ohne daß anschließend Verklumpung auftreten würde, wie dies bei Verwendung von Hydraten auftritt.

So können die wasserfreien Produkte mit Flüssigkeiten auf anorganischer oder organischer Basis getränkt werden und dienen dann als deren Träger, bleiben aber als solche trocken und rieselfähig.

Die Entwässerung der kristallinen Hydrate kann in jeder industriellen Mischanlage, z.B. in einem beheizten Lödigemischer, erfolgen, bevorzugt wird jedoch die Wirbelschichttrocknung, da dort der mechanische Oberflächenabrieb gering gehalten werden kann, und infolge der kurzen Trockenzeiten, die z.B. bei einer Fluidisierungslufttemperatur von etwa 250°C nicht mehr als etwa 30 min betragen.

### Herstellungsbeispiel:

In einem Lödige-Mischer wurde aus 10 kg Trinatriumcitratdihydrat bei einer Produkttemperatur von 150-190°C innerhalb von 60 min etwa 1,25 kg Wasser entfernt.

### Anwendungsbeispiel 1:

Das im Herstellungsbeispiel erhaltene Produkt (8,75 kg) wurde bei Raumtemperatur mit 1 kg 60 %igem H₂O₂ vermischt, das nach etwa 30 min vollständig eingesaugt war. Das trockene Produkt hatte einen Gehalt von 6 % H₂O₂ (2,67 % Aktivsauerstoff).

Nach 6wöchiger Lagerzeit war das Produkt weiterhin rieselfähig ohne Abnahme des Peroxidgehalts.

### Anwendungsbeispiel 2

1 kg Trinatriumcitrat-Anhydrat wurde mit 100 g DEHYPON LS 54 (Tensid der Fa. Henkel) rund 15 Minuten vermischt und es entstand ein einwandfrei rieselfähiges Produkt.

### Anwendungsbeispiel 3

1 kg Trinatriumcitrat-Anhydrat wurde mit 10 g DEHYDOL LS4 (Tensid der Fa. Henkel) rund 15 min vermischt und es entstand ein einwandfrei rieselfähiges Produkt.

### Vergleich:

Anwendungsbeispiel 3 wurde mit Trinatriumcitratdihydrat wiederholt; das Produkt war nicht rieselfähig.

### Anwendungsbeispiel 4

1 kg Trinatriumcitrat-Anhydrat wurde mit 1 kg Citronensäure-Anhydrat vermischt und einem Lagertest von 6 Wochen unterzogen. Nach dieser Zeit war das Produkt noch einwandfrei rieselfähig.

### Vergleich:

Anwendungsbeispiel 4 wurde mit Trinatriumcitratdihydrat wiederholt; nach 6 Wochen war das Produkt vollständig verklumpt.

### Löslichkeitsvergleich:

In der folgenden Tabelle werden die Auflösungszeiten in Minuten von einerseits wasserfreiem Trinatriumcitrat und anderseits Trinatriumcitratdihydrat gleicher Korngrößenverteilung in Wasser bei 25°C verglichen:

| Zusatzmenge Salz (Gew.-%) | wasserfrei | Dihydrat |
|---|---|---|
| 10 | 0,46 | 1,05 |
| 30 | 0,59 | 2,14 |

Das wasserfreie Salz löst sich somit erheblich rascher auf.

## Patentansprüche

1. Wasserfreies Trinatriumcitrat mit gegenüber einer kristallinen Hydratform unveränderter Struktur.

2. Wasserfreies Trinatriumcitrat gemäß Anspruch 1 als Träger von anorganischen und/oder organischen Substanzen, insbesondere nach Tränken mit Flüssigkeiten.

3. Verfahren zur Herstellung von wasserfreiem Trinatriumcitrat gemäß Anspruch 1 aus einem entsprechenden kristallinen Hydrat, dadurch gekennzeichnet, daß das Hydrat, insbesondere das Dihydrat, unter Bewegung getrocknet wird.

4. Verfahren nach Anspruch 3, dadurch gekennzeichnet, daß das Hydrat in einer Wirbelschicht in einem Heißluftstrom, insbesondere bei einer Hydrattemperatur von bis zu etwa 200°C, vorzugsweise 150-190°C, getrocknet wird.

## Claims

1. Anhydrous trisodiumcitrate, the structure of which is unchanged as compared to a crystalline hydrate mode.

2. The anhydrous trisodiumcitrate according to claim 1 as a carrier for inorganic and/or organic substances, in particular after being impregnated with liquids.

3. A process for the production of anhydrous trisodiumcitrate according to claim 1 from a corresponding crystalline hydrate, characterised in that the hydrate, in particular the dihydrate, is dried while being maintained in motion.

4. A process according to claim 3, characterised in that the hydrate is dried in a fluidized bed in a stream of heated air, in particular at a hydrate temperature of up to about 200 °C, preferably 150-190 °C.

## Revendications

1. Tricitrate de sodium anhydre dont la structure est la même que celle de l'hydrate cristallin.

2. Tricitrate de sodium anhydre selon la revendication 1 comme support de matières inorganiques et/ou organiques, en particulier après d'être imprégné avec des liquides.

3. Procédé de préparation de tricitrate de sodium anhydre selon la revendication 1 à partir d'un hydrate cristallin correspondant, caractérise en ce que l'hydrate, en particulier le dihydrate, est séché sous mouvement.

4. Procédé de préparation selon la revendication 3, caractérisé en ce que l'hydrate est séché dans un lit fluidisé dans un courant d'air chaud, en particulier à une température d'hydrate jusqu'à 200°C environ, préférablement 150 à 190°C.
